Europäisches Patentamt

European Patent Office

Office européen des brevets

(11) Publication number: **0 145 373 B1**

# EUROPEAN PATENT SPECIFICATION

(45) Date of publication of patent specification: **25.03.92**

(51) Int. Cl.⁵: **G01N 33/574**, G01N 33/577, C07K 3/02, C12N 15/00, C07K 15/06, C07K 15/24, C07K 3/28

(21) Application number: **84308081.3**

(22) Date of filing: **21.11.84**

The file contains technical information submitted after the application was filed and not included in this specification

(54) **Purification of cancer-associated protein and preparation of antibody thereto.**

(30) Priority: **23.11.83 US 554439**
**21.11.84 PCT/US84/01932**

(43) Date of publication of application:
**19.06.85 Bulletin 85/25**

(45) Publication of the grant of the patent:
**25.03.92 Bulletin 92/13**

(84) Designated Contracting States:
**AT BE CH DE FR GB IT LI LU NL SE**

(56) References cited:
**GB-A- 2 067 286**

**CHEMICAL ABSTRACTS, vol. 100, no. 9, 27 Feb 1984, Columbus, OH (US); D.E.SCHUMM et al., p. 415, no. 65997h**

**CHEMICAL ABSTRACTS, vol. 98, no. 5, 31 Jan 1983, Columbus, OH (US); D.E.SCHUMM et al., p. 480, no. 32357m**

**NATURE, vol. 256, 07 August 1975, Basing-stoke (GB); D.H.SCHUMM et al., pp. 508-509**

(73) Proprietor: **THE OHIO STATE UNIVERSITY RE-SEARCH FOUNDATION**
**1314 Kinnear Road**
**Columbus, Ohio 43212(US)**

(72) Inventor: **Webb, Thomas E.**
**2050 Nayland Rd.**
**Columbus, OH 43220(US)**
Inventor: **Schumm, Dorothy E.**
**1136 Neil Avenue**
**Columbus, OH 43201(US)**
Inventor: **Hanausek-Walaszek, Margaret**
**3063 Jersey Drive**
**Columbus, OH 43204(US)**

(74) Representative: **Cole, David John**
**Executive Liaison Services 54 Templemere**
**Weybridge Surrey KT13 9PB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 145 373 B1

CHEMICAL ABSTRACTS, vol. 82, no. 21, 26 May 1975, Columbus, OH (US); D.E.SCHUMM et al., p. 412, no. 137333y

CHEMICAL ABSTRACTS, vol. 98, no. 15, 11 April 1983, Columbus, OH (US); J.RACEVSKIS et al., p. 266, no. 121630n

CHEMICAL ABSTRACTS, vol. 98, no. 17, 25 April 1983, Columbus, OH (US); H.R.SOULE et al., pp. 412-413, no. 141420x

BIOLOGICAL ABSTRACTS, vol. 75, 1983, Philadelphia, PA (US); K.SEGAWA et al., p. 5540, no. 53951

CANCER RESEARCH, vol. 42, 1982; D.H.SCHUMM et al., pp. 4964-4969

## Description

This invention relates to a method for determining the presence in a mammal of a cancer-associated antigen, an antibody preparation, and a protein preparation for use in producing the antibody preparation.

In spite of improved treatments for certain forms of cancer, it is still the second leading cause of death in the United States. Since the chance for complete remission of cancer is, in most cases, greatly enhanced by early diagnosis, it is very desirable that physicians be able to detect cancers before a substantial tumor develops. Also, in cases where the primary tumor has been substantially removed by surgery or destroyed by other means, it is important that the physician be capable of detecting any trace of cancer in the patient (either in the form of residues of the primary tumor or of secondary tumors caused by metastasis), in order that the physician can prescribe appropriate subsequent treatment, such as chemotherapy.

The quantities of cancer cells which must be detected for early diagnosis or following removal or destruction of the primary tumor are so small that the physician cannot rely upon physical examination of the cancer site; moreover, in many cases the cancer site is of course not susceptible of direct visual observation and it is almost always impractical to detect secondary tumors by visual observation, since it is not possible to predict exactly where they are likely to occur. Accordingly, sensitive tests have to rely upon detection of cancer-associated materials, usually proteins, present in body fluids of patients who have, or about to develop, small numbers of cancer cells in their bodies. Diagnostic materials for detection of at least two cancer-associated proteins are already available commercially. Tests for alpha-fetoprotein are used to detect primary liver cancer and teratocarcinoma in humans. Similarly, carcinoembryonic antigen is used for diagnosis of cancers of the digestive system. Unfortunately, such tests are only applicable to a narrow range of cancer types, and therefore these tests suffer not only from the disadvantage that other types of cancer may be missed but also from the disadvantage that the narrow applicability of the tests means that it may be necessary to run multiple tests on a single patient for diagnostic purposes, a procedure which not only increases the expense of the diagnostic testing but also increases the risk that one or other of the test may give a false positive result. Accordingly, there is a need for a single chemical test able to detect the presence of very small amounts of cells of a wide variety of different cancers.

It is already known that serum from the blood of animals suffering from a wide variety of cancers contains a protein (hereinafter referred to as "cancer marker protein") having a molecular weight of approximately 60,000 and having the capacity to increase the release of ribonucleic acid (RNA) form cell nuclei; see:

Schumm et al, Apparent Transformed Cell-dependent Proteins in Blood Plasma, Proc. Am. Assoc. Cancer Res., 22:79 (1981);

Schumm and Webb, Differential Effect of Plasma Fractions from Normal and Tumor-Bearing Rats on Nuclear RNA Restriction, Nature (London), 256:508-509 (1975);

Schumm and Webb, Modification of Nuclear Restriction In Vitro by Plasma from Tumor-Bearing Animals, J. Natl. Cancer Inst., 54:123-128 (1975);

Schumm and Webb, Apparent Transformed Cell-Dependent Proteins in Blood Plasma, Proc. Am. Assoc. Cancer Res. 22:79 (1981).

Thus, testing for this cancer marker protein is a potential, sensitive method for the detection of a wide variety of cancers in humans and other mammals. Unfortunately, the only method of detecting this cancer marker protein described in the aforementioned papers involves assaying the ability of serum protein from the patient being tested to release RNA from rat liver nuclei; see:

Schumm et al, Changes in Nuclear RNA Transport Incident to Carcinogenesis, Eur. J. Cancer, 13:139-147 (1977);

Schumm et al, Cytosol-Modulated Transport of Messenger RNA from Isolated Nuclei Cancer Res., 33:1821-1828 (1973).

Although, as shown in the aforementioned papers, this test is capable of detecting cancer in patients, the assaying method used is not suitable for routine use by medical technicians. Accordingly, there is a need for a simpler, less costly test for detection of this cancer marker protein.

Further work on detection of this cancer-associated antigen is described in:

Schumm and Webb, Putative Transformation-dependent proteins in the blood plasma of tumor-bearing rats and cancer patients, Cancer Research, 42: 4964-69 (December 1982).

This paper shows that the cancer-associated antigen is present in the blood of rats exposed to a carcinogen and in the blood of humans suffering from a wide variety of cancers. Again, detection of the antigen was effected by the RNA-releasing test. Furthermore, normal pregnancy, a non-malignant condition in which fetal proteins are found in the circulation, also gave positive results.

One method often used for detection of proteins and other antigens is radioimmunoassay. In this technique, a sample of the material to be assayed is mixed with a known quantity of a radioactive-labeled form of the antigen to be measured. The resultant mixture is then treated with antibody to the antigen being assayed, thereby forming an antigen-antibody complex. The degree of radioactivity of this complex depends upon the amount of the appropriate antigen in the sample being assayed and thus, by measuring the radioactivity of the complex, the quantity of the antigen in the sample may be determined.

Radioimmunoassay techniques are suitable for routine use by medical technicians and indeed are already used for assay of a variety of antigens in various body fluids. Thus, radioimmunoassay is potentially an attractive technique for testing for the cancer marker protein. However, a radioimmunoassay technique requires the prior preparation of an antibody which is specific to the antigen being assayed and the aforementioned papers do not describe any method for the preparation of such an antibody. Moreover, to prepare an antibody specific to the cancer marker protein, it is necessary to prepare the cancer marker protein in a relatively pure form, since the antibodies have to be prepared by injecting the cancer marker protein into an animal and recovering serum therefrom, and any impurities in the cancer marker protein may give rise to corresponding antibodies to those impurities, thereby interfering with the accurate assay of the cancer marker protein in the radioimmunoassay test.

Another method for the detection of proteins and other antigens is known as an ELISA assay. In this assay, the walls of wells in a culture plate are coated with an antibody to the antigen to be assayed and the wells are then washed. A specimen of the material to be tested is placed in the coated wells, incubated for a period sufficient to allow any antigen present to react with the antibody on the walls, and after incubation the wells are again washed out. Next, there is added to the wells a conjugate of the antibody and horseradish peroxidase, the plates are incubated and washed out, and a mixture of hydrogen peroxide in a buffer and 2,2'-AzinoDi(3-Ethylbenzthiazoline sulfonic Acid) (ABTS) and the plates are allowed to develop for ten minutes at room temperature. The reaction was then stopped by the addition of 2mM sodium azide solution. If the antigen being assayed (i.e. that specific to the antibody) is not present in the test material, no color will be formed in the wells of the plates because the peroxidase enzyme will have been washed away. If, however, the antigen being assayed is present in the test material immunological reactions will cause the enzyme to adhere to the walls of the wells and this enzyme will cause formation of a color with the hydrogen peroxide/ABTS mixture. Thus, the formation of color at the end of the test is indicative of the presence of the antigen. Like radioimmunoassay, this assay technique requires the preparation of an antibody specific to the antigen being assayed and the preparation of this antibody requires purification of the antigen.

The present invention provides a method for determining the presence in a mammal of a cancer-associated antigen, this method being based upon that described in the papers referred to above. This invention also provides an antibody preparation for use in this method, and a protein preparation useful for preparing this antigen.

Accordingly, this invention provides a method for determining the presence in a mammal of a cancer-associated antigen, the method comprising:

producing antibodies to the antigen;

contacting the antibodies with biological material of the mammal; and

determining the presence of a reaction product formed between the antibodies and the antigen, the antigen being a phosphoprotein having the following characteristics:

(a) having a molecular weight of approximately 60,000; and

(b) having the capacity to liberate ribonucleic acid from cell nuclei,

characterized in that the antigen has the following further characteristics:

(c) not being precipitated by 30% saturated aqueous ammonium sulfate solution at 25°C;

(d) being precipitated from aqueous solution by 3.3% streptomycin sulfate;

(e) having substantially no autophosphorylation activity but being phosphorylated with adenosine triphosphate in the presence of an exogenous protein kinase;

(f) having the capacity to liberate ribonucleic acid from the cell nuclei;

(g) not being present in the maternal blood of noncancerous normal pregnant mammals of the species in which the protein is being determined.

The term "RNA-releasing activity" as used herein refers to activity determined by the following procedure. Male Sprague-Dawley rats weighing approximately 250 grams are fasted for 18 hours. For the preparation of cytosol, the rat livers are dissected out, homogenized in a volume of 0.25 M sucrose-TMK buffer which is twice the wet weight (milliliters/gram) of the livers.

The TMK buffer used has the following composition:

50 mM tris (hydroxy methyl amino) methane hydrochloride 25 mM potassium chloride
2.5 mM magnesium chloride
This buffer has a pH of 7.5.

The homogenate is centrifuged at 100,000g for 90 minutes and the supernatant removed and dialyzed overnight against TMK buffer.

For the preparation of nuclei, rats are injected with 50 micro curies of [6-$^{14}$C] orotic acid (specific activity 323 micro curies/milligram; this material is available from New England Nuclear Corporation, Boston, Massachusetts) as described in the aforementioned paper in Cancer Res., 33:1821-1828 (1973). After a 30 minute prelabelling, the rat livers are dissected out, homogenized in a volume of 2.3M sucrose 3.3mM calcium acetate aqueous solution which is 15 times the weight of the liver. This homogenate is centrifuged at 34,000g for 60 minutes at 4°C. The nuclear pellet is washed with a solution containing 1M sucrose/lmM calcium acetate and resuspended in the same solution.

The cytosol remaining after dialysis is then used to prepare a cell-free system containing 5 x 10$^6$ prelabeled nuclei per milliliter of medium containing 5 milligram of dialyzed cytosol protein per milliliter and having the following additional components:

50mM Tris-HCl (pH 7.5)
25mM potassium chloride
2.5mM magnesium chloride
0.5mM calcium chloride
0.3mM manganese chloride
5.0mM sodium chloride
2.5mM phosphoenol pyruvate
35 units/milliliter of pyruvate kinase
2.5mM sodium dihydrogen phosphate
5.0mM spermidine
2.0mM dithiothreitol
2.0mM adenosine triphosphate

300 microgram/milliliter of low molecular weight yeast RNA.

An aliquot of up to 100 microliters of plasma or 200 microliters of a column fraction containing the cancer marker protein is added to 1 milliliter of the cell-free medium and incubated at 30°C for 30 minutes. The nuclei are removed by centrifugation, the resulting supernatant liquor separated and the RNA and protein precipitated therefrom with a 5% aqueous solution of trichloroacetic acid. The resultant precipitate is washed in ethanol, dissolved in solubilizer and counted in liquid scintillant. The preferred solubilizer is "Unisol" and the preferred scintillant "Unisol Complement", both available from Isolab, Inc., Akron, Ohio. A control is treated in the same manner, except of course, that none of the specimen being tested is added thereto. One unit of activity represents an increase in the count of 1% of the total nuclear counts and, for obvious reasons, results are normally expressed as units of activity per milligram of protein added to the test material.

This invention also provides an antibody preparation substantially free of antibodies to normal plasma fraction and characterized in that it is precipitated by 35% saturated aqueous ammonium sulfate solution, and is capable of forming a conjugate with a cancer-associated phosphoprotein derived from a mammal and having the characteristics:

(a) not being precipitated by 30% saturated aqueous ammonium sulfate solution at 25°C;
(b) having a molecular weight of approximately 60,000;
(c) being precipitated from aqueous solution by 3.3% streptomycin sulfate;
(d) having substantially no autophosphorylation activity but being phosphorylated with adenosine triphosphate in the presence of an exogenous protein kinase;
(e) having substantially no protein kinase activity;
(f) having the capacity to liberate ribonucleic acid from cell nuclei;
(g) being substantially free of albumin; and
(h) absent from the maternal blood of non-cancerous pregnant mammals of the species from which the protein is derived

the antibody preparation forming a visible precipitate with the cancer marker protein when they are diffused toward one another in agar gel, but the antibody preparation not being capable of forming a conjugate with the 25K protein fraction from human plasma nor the 35K protein fraction from rat plasma.

Finally, this invention provides a protein preparation comprising a cancer-associated phosphoprotein derived from a mammal and having the following characteristics:

(a) having a molecular weight of approximately 60,000; and
(b) having the capacity to liberate ribonucleic acid from cell nuclei,

characterized in that the phosphoprotein has the further following characteristics:

(c) not being precipitated by 30% saturated aqueous ammonium sulfate solution at 25°C;
(d) being precipitated from aqueous solution by 3.3% streptomycin sulfate;
(e) having substantially no autophosphorylation activity but being phosphorylated with adenosine triphosphate in the presence of an exogenous protein kinase;
(f) having substantially no protein kinase activity;
(g) being substantially free of albumin; and
(h) absent from the maternal blood of non-cancerous pregnant mammals of the species from which the protein is derived

the protein preparation having an RNA-releasing activity of at least about 10 units per milligram of total protein in the protein preparation.

Fig. 1 is a graph showing the distribution of RNA-releasing activity from human cancer patients and healthy individuals obtained by the procedure described in Example 1 below; and

Fig. 2 is a graph, similar to Fig. 1, but showing the RNA-releasing activity distribution from pregnant women.

As already mentioned, this invention provides a protein preparation comprising a purified form of the aforementioned cancer marker protein and having an RNA-releasing activity of at least 10 units per milligram of total protein in the preparation. The RNA-releasing activity of the instant protein preparation is desirably about 10 units per milligram of total protein in the preparation. Typically in the purification of the cancer marker protein from humans, the dialyzed protein from the second step of the purification process will have an activity of about 0.84 units per milligram of protein, the 60,000 molecular weight fraction from the third step of the purification process will have an activity of about 2.66 units per milligram of total protein and the final material will have an activity of about 20 units per milligram of protein. In the purification of the cancer marker protein from rats, the dialyzed protein from the second step will have an activity of about 1.4 units per milligram, the 60,000 molecular weight fraction from the third step will have an activity of about 8.1 units per milligram and the final material will have an activity of about 71 units per milligram. It should be noted that one cannot correlate the increase in specific activity of the protein during the various stages of the purification process directly with the degree of purification affected, since it appears that the cancer marker protein does lose some of its RNA-releasing activity during the purification process, and especially in the final step thereof. It is necessary that the protein preparation be substantially free of albumin since albumin is strongly immunogenic and thus any albumin in the instant protein preparation would produce antibody serum heavily contaminated with albumin antibodies.

Although the characteristics are already specified are believed to define the instant cancer marker protein uniquely, it should be noted that various other properties of the cancer marker protein have been observed and further characterize this protein. The cancer marker protein is fairly stable, retaining full biochemical activity and showing little or no change in size during storage in plasma at -20°C for at least three years. However, the protein is heat-labile, being completely destroyed by heating to 65°C for 10 minutes. The protein has little or no single strand ribonuclease activity and electrophoresis on sodium dodecyl sulfatepolyacrylamide gel suggests that it consists of a single polypeptide chain since a single band appears in the 60,000 molecular weight region.

As already mentioned, the first stage in the instant method for preparing the purified cancer marker protein is separating from the serum of a mammal suffering from cancer the fraction of serum protein which is not precipitated by 30% saturated aqueous ammonium sulfate solution. Since the purpose of the ammonium sulfate precipitattion step is to remove low molecular weight material, one can use the whole fraction precipitating between 30% and 100% saturation. However, in general it is preferred to use the fraction precipitating between 30% and 60% saturation, since this fraction contains substantially all the cancer marker protein.

The instant cancer marker protein preparations can be isolated from both humans and other mammals, and the protein preparations from different species appear to be generally similar in molecular weight and other chemical properties. However, the cancer marker proteins from different species are not immunologically equivalent e.g. an antibody to the rat cancer marker protein does not cross-react with a human cancer marker protein. Thus, when the purified cancer marker protein preparation is to be used for production of antibodies for diagnostic purposes, it is necessary to begin the preparation process with plasma from the species in which the diagnosis is to be used.

The second, dialysis step of the instant purification process is desirably conducted at a pH in the range of about 7 to about 8. In a particularly preferred embodiment of the invention, the dialysis is conducted by dispersing the plasma protein fraction in a tris(hydroxymethylamino)-methane/potassium chloride/magnesium chloride buffer having a pH of about 7.5 and dialyzing the resultant protein solution against the same buffer. Overnight dialysis normally provides sufficient purification in this step of the instant process.

In the third step of the instant purification process, the fraction of the protein having a molecular weight of about 60,000 is separated. Although, as already noted, the molecular weight of the cancer marker protein is about 60,000, the fractions eluting in the molecular weight range of 55,000 to 70,000 should usually be collected to ensure reasonably complete recovery of the protein. Separation of the 60,000 molecular weight fraction of protein can be achieved using centrifugation, but is is preferred that this separation be effected by chromatography of the dialyzed protein from the second step of the instant process on a molecular seive solid phase. Those skilled in the art will be aware of a variety of molecular sieve materials which can be used for this purpose; the preferred molecular sieve material is the cross-linked resin available commercially under the trade name Sepharose CL-6B. The liquid phase in the chromatography process is preferably the same TMK buffer used in the aforementioned preferred embodiment of the dialysis step. However, following the chromatography step the purified cancer marker protein is still contaminated by a considerable amount of serum albumin (molecular weight about 68,000). Accordingly, to remove this impurity it is preferred that the instant purification process includes the step of treating the 60,000 molecular weight protein fraction obtained from the third step to remove albumin therefrom. The preferred method for removal of albumin without causing any deleterious effects on the cancer marker protein itself is chromatography on a solid medium capable of absorbing albumin, and a preferred medium for this purpose is that available commercially under the trade name CM Affi-Gel Blue (obtainable from Bio-Rad Lab., Richmond, California).

It should be noted that, once one batch of the purified cancer marker protein has been isolated and a serum containing antibodies thereto produced, the purification of later batches of cancer marker protein can be enhanced by treating the purified cancer marker protein isolated from the molecular sieve chromatography step (before or after removal of albumin therefrom) with the antibody-containing serum. As those skilled in the art are aware, this treatment will cause precipitation of a cancer marker protein/antibody complex which can then be separated, for example by centrifugation, and broken down by methods familiar to those skilled in the art to yield the purified cancer marker protein and the antibody; for example the protein/antibody complex may be broken down on a column of protein A agarose (sold by BRL, Gaithersburg, MD; directions for use of this material are supplied by the manufacturer).

As already mentioned, to produce a serum containing antibodies to the cancer marker protein, the purified protein is introduced into the bloodstream of a mammal and allowed to remain in the mammal for at least seven days, blood is removed from the mammal and serum prepared from this blood. To produce high levels of antibodies to the cancer marker protein, it is preferred that the purified protein be introduced into the bloodstream of the mammal together with an immunostimulating adjuvant, complete or incomplete Freund's adjuvant being efficacious for this purpose. The purified protein is desirably introduced into the mammal by intradermal injection, and a preferred administration routine comprises a primary immunization with the purified cancer marker protein and immunostimulating adjuvant, followed by a second immunization with the protein preparation and an immunostimulating adjuvant at least seven days after the primary immunization, the blood being removed from the mammal at least three days after the second immunization. A convenient mammal for use in the process is a rabbit. In order to produce an antibody-containing serum which is specific for the cancer marker protein, the serum prepared from the blood of the immunized mammal is absorbed with normal plasma fraction, thereby eliminating all antibodies produced to normal components of plasma which have carried through the purification process, and producing a purified antibody preparation which is specific to the cancer marker protein. (Even after the instant purification process, the cancer marker protein still contains traces of immunogenic impurities, so that the antibody produced by passage through the mammal is polyclonal. The absorption with normal plasma fraction removes the antibodies to normal plasma components, thereby ensuring that the antibodies are specific for the cancer marker protein and will not react with normal plasma components to give false positive results when the antibody is used for diagnostic tests as described below. The antibody produced after adsorption with normal plasma fraction gives a single precipitin line when reacted with the cancer marker protein and thus appears to be monospecific for the cancer marker protein.

The antibody thus prepared may be used to test for the presence of cancer marker protein in serum from suspected cancer patients by a variety of conventional tests. Thus, the antibody may be used in a radioimmunoassay, the technique for which has already been described above. The radio-labeled cancer marker protein needed for this test may be prepared from the instant cancer marker protein preparation using a procedure described by Ganguli and Hunter in Radioimmune Assay Methods (European Workshop, 1970). This radio-labeling technique involves mixing sodium iodide containing $^{125}$I phosphate buffer having a pH of 7.5, the cancer marker protein and chloramine T, incubating for 30 seconds and stopping the labeling reaction with metabisulfate and potassium iodide. The radio-labeled cancer marker protein can be separated from the reaction mixture by chromatography on Sephadex G-50.

Alternatively, the instant antibody may be used in an ELISA assay. The preferred technique for carrying out such an assay with the instant antibody is as follows. The instant antibody is purified using a 35% solution of ammonium sulfate by the standard precipitation/resolubilization technique and part of the antibody is then conjugated to horseradish peroxidase by the conventional techniques used in ELISA assays. Conveniently, conjugation of the horseradish peroxidase to the antibody is effected using 1-fluoro-2,4-dinitrobenzene (FDNB). A 1% solution of FDNB in absolute ethanol is mixed with the horseradish peroxidase in a bicarbonate buffer, and the resultant mixture incubated. After this incubation, ethylene glycol is added to the solution, which is then dialyzed against the bicarbonate buffer to yield horseradish peroxidase aldehyde. The antibody-containing serum fraction obtained by salt precipitation and dialysis of antibody-containing serum is added to the horseradish peroxidase aldehyde solution. After reaction is complete, sodium borohydride is added to the solution, which is then dialyzed overnight against a phosphate-buffered saline solution. The dialyzed solution is chromatographed on Sephadex G-200 gel; the first protein peak eluting from the column is the desired horseradish peroxidase/antibody conjugate. A base coat of the unconjugated portion of the antibody is deposited in the wells of a Dyntech Immulon II microelisa plate by allowing 100 microliters containing several micrograms of the antibody to remain in each well overnight at 4°C. Thereafter, the wells are washed three times and then 100 microliters of test serum from the patient added to each well (for obvious reasons, the tests are normally run in duplicate). If the cancer marker protein is present in the test serum, it will conjugate to the antibody already stuck to the walls of the cell and will thus deposite the cancer marker protein on the walls of the well. To permit the conjugation between the antibody on the walls of the cell and any cancer marker protein in the test serum, the plates are incubated at 37°C for one hour and, at the end of this time, washed three times. Next, 100 microliters containing several micrograms of the antibody conjugated to the peroxidase are added to each well and the plates again incubate at 37°C for one hour. As already described, if the test serum contains cancer marker protein, this cancer marker protein will have been deposited on the walls of the well and, upon addition of the antibody/peroxidase conjugate, this conjugate will become attached to the cancer marker protein, thus depositing the conjugate on the walls of the well. If, however, no cancer marker protein was present in the test serum, the antibody/peroxidase conjugate will not adhere to the walls of the wells. Following the incubation with the antibody/peroxidase conjugate, the wells are washed five times, thus removing any conjugate which is not adhered to the walls of the wells. Finally, a substrate for the enzyme comprising hydrogen peroxide in a buffer and ABTS is added to the wells and allowed to develop for ten minutes at room temperature, after which time 20 microliters of a 2mM sodium azide stop solution are added to each well The results are read on a microelisa reader. Obviously, if cancer marker protein was present in the test serum and the antibody/peroxidase conjugate was deposited on the walls of the well, color will be developed as the peroxidase in the deposited conjugate acts on the substrate, whereas if no cancer marker protein was present in the test serum there will be no conjugate present on the walls of the well and no color will be developed.

The following examples are now given, though by way of illustration only, to show details of particularly preferred reagents and techniques used in the instant processes.

Example 1

This example illustrates the instant process for purification of the cancer marker protein.

Plasma obtained from ten human patients having cancers at eight different sites was pooled, then fractionated with ammonium sulfate. The protein precipitating between 30 and 50% saturation of the aqueous ammonium sulfate solution, which fraction was found to contain all the RNA-releasing activity, was dissolved in approximately 5ml of the aforementioned TMK buffer, then dialyzed overnight against the same buffer. An aliquot of the dialyzed solution containing approximately 150mg. of total protein was applied to a 1.5 x 90cm. column of the molecular sieve resin, Sepharose CL-6B. The column was eluted with the aforementioned TMK buffer and 3ml fractions were collected. A 200 microliter aliquot of each even-numbered fraction was assayed for RNA-releasing activity as described above.

For control purposes, an exactly similar procedure was followed with pooled plasma from 10 persons who were not suffering from cancer.

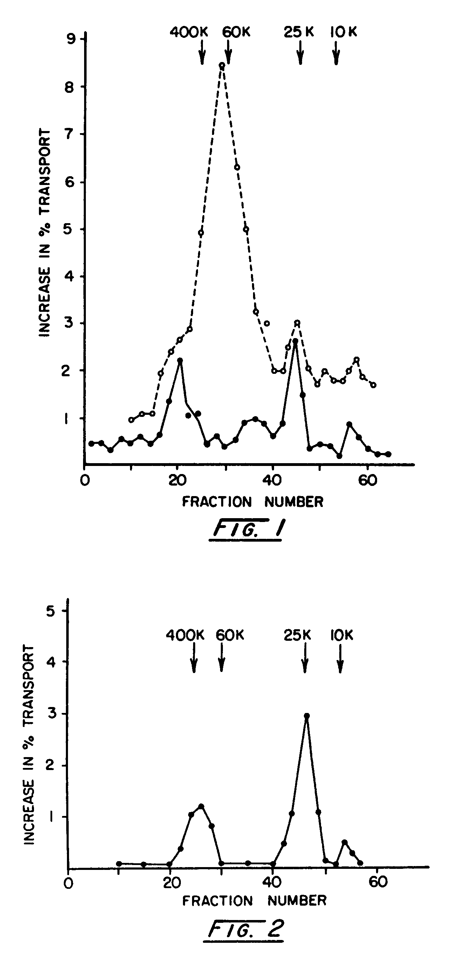

The results of the assay are shown in Fig. 1, in which the broken line shows the results obtained from the cancer patients and the continuous line shows the results obtained from the healthy individuals. It will be seen from Fig. 1 that the two profiles are completely different, the cancer patients showing a very strong peak at around 60,000 molecular weight, which is completely absent from the profile of the normal patients. At about fraction number 30, the protein from the cancer patients was showing an increased activity in the assay of approximately 8.5%, equal to an activity of 10 units per milligram of protein in the aliquot.

Further purification of the fractions eluting from the molecular sieve column at a molecular weight of 55,000-70,000 was effected by a further chromatography on a 1.0 x 10.0cm column of CM Affi-Gel Blue, in order to remove albumin from the purified cancer marker protein. A sample containing 25.0mg of protein was suspended in the aforementioned TMK buffer, loaded onto the column and eluted with 0.4M potassium chloride-TMK buffer. Dialysis of eluate and electrophoresis on sodium dodecylsulfate-polyacrylamide gel confirmed that this treatment substantially completely removed albumin from the purified cancer marker preparation. The electrophoretograms produced only a single band corresponding to the cancer marker protein, suggesting that this protein is composed of a single polypeptide chain.

Since it is known that certain cancer-associated proteins are oncofetal proteins, and since earlier research had indicated that the plasma from pregnant women showed an abnormally high activity in the RNA-releasing activity test, plasma from a pregnant woman was subjected to the purification process already described. The profile obtained is shown in Fig. 2. From this figure, it will be seen that the 60,000 molecular weight peak associated with the cancer marker protein is completely absent from the plasma of the pregnant woman and the the RNA releasing activity of the serum is associated with a peak at a molecular weight of 25,000. Accordingly, it appears that the cancer marker protein is completely absent from the plasma of pregnant women and thus pregnancy should not interfere with the instant radioimmunoassay test.

Example 2

This example illustrates the instant process for the preparation of an antibody.

The purified cancer marker protein prepared as described in Example 1 was dissolved in normal saline at a concentration of 1.5 milligram of protein per milliliter. 1 ml. of this normal saline solution was then emulsified with 1 ml. of complete Freund's adjuvant using an omni-mixer with a microcontainer immersed in an ice-water bath. The 2 ml. of emulsion thus produced was injected into multiple intradermal sites on the shaved backs of young adult New Zealand white rabbits. This primary immunization was followed by the second immunization approximately 30 days later, the second immunization being performed by the same method as the first except that incomplete Freund's adjuvant was substituted for complete Freund's adjuvant. The animals were bled 1, 2 and 3 weeks after the second immunization and immunodiffusion analyses were conducted to determine the potency and specificity of the antisera obtained. The antisera prepared were absorbed with normal plasma fraction to produce an antibody specific to the cancer marker protein.

**Claims**

1. A method for determining the presence in a mammal of a cancer-associated antigen, the method comprising:

   producing antibodies to the antigen;

   contacting the antibodies with biological material of the mammal; and

   determining the presence of a reaction product formed between the antibodies and the antigen, the antigen being a phosphoprotein having the following characteristics:

   (a) having a molecular weight of approximately 60,000; and

   (b) having the capacity to liberate ribonucleic acid from cell nuclei,

   characterized in that the antigen has the further following characteristics:

   (c) not being precipitated by 30% saturated aqueous ammonium sulfate solution at 25°C;

   (d) being precipitated from aqueous solution by 3.3% streptomycin sulfate;

   (e) having substantially no autophosphorylation activity but being phosphorylated with adenosine triphosphate in the presence of an exogenous protein kinase;

   (f) having the capacity to liberate ribonucleic acid from cell nuclei;

   (g) not being present in the maternal blood of non-cancerous normal pregnant mammals of the species in which said protein is being determined.

2. A method according to claim 1, characterized in that the presence of the reaction product is determined by radioimmunoassay.

3. A method according to claim 1, characterized in that the presence of the reaction product is determined by ELISA assay.

4. A method according to any one of the preceding claims characterized in that the biological material is the plasma of the mammal.

5. A method according to any one of claims 1 to 3, characterized in that the antibodies are derived from a second mammal immunized with the antigen.

6. An antibody preparation substantially free of antibodies to normal plasma fraction, characterized in that it is precipitated by 35% saturated aqueous ammonium sulfate solution and in that it is capable of forming a conjugate with a cancer-associated phosphoprotein derived from a mammal and having the following characteristics:

   (a) not being precipitated by 30% saturated aqueous ammonium sulfate solution at 25°C;

   (b) having a molecular weight of approximately 60,000;

   (c) being precipitated from aqueous solution by 3.3% streptomycin sulfate;

(d) having substantially no autophosphorylation activity but being phosphorylated with adenosine triphosphate in the presence of an exogenous protein kinase;

(e) having substantially no protein kinase activity;

(f) having the capacity to liberate ribonucleic acid from cell nuclei;

(g) being substantially free of albumin; and

(h) absent from the maternal blood of non-cancerous pregnant mammals of the species from which the protein is derived,

the antibody preparation forming a visible precipitate with the cancer-associated phosphoprotein when they are diffused toward one another in agar gel, but the antibody preparation not being capable of forming a conjugate with the 25K protein fraction from human plasma nor the 35K protein fraction from rat plasma.

7. A protein preparation comprising a cancer-associated phosphoprotein derived from a mammal and having the following characteristics:

(a) having a molecular weight of approximately 60,000; and

(b) having the capacity to liberate ribonucleic acid from cell nuclei,

characterized in that the phosphoprotein has the following further characteristics:

(c) not being precipitated by 30% saturated aqueous ammonium sulfate solution at 25°C;

(d) being precipitated from aqueous solution by 3.3% streptomycin sulfate;

(e) having substantially no autophosphorylation activity but being phosphorylated with adenosine triphosphate in the presence of an exogenous protein kinase;

(f) having substantially no protein kinase activity;

(g) being substantially free of albumin; and

(h) absent from the maternal blood of non-cancerous pregnant mammals of the species from which the protein is derived,

the protein preparation having an RNA-releasing activity of at least about 10 units per milligram of total protein in the protein preparation.

**Revendications**

1. Procédé pour déterminer la présence, chez un mammifère, d'un antigène associé au cancer, le procédé comprenant les étapes consistant à :

- produire des anticorps vis-à-vis de l'antigène,

- mettre en contact les anticorps avec de la matière biologique du mammifère, et

- déterminer la présence d'un produit réactionnel formé entre les anticorps et l'antigène, l'antigène étant une phosphoprotéine et ayant les caractéristiques suivantes :

(a) il a un poids moléculaire d'approximativement 60 000, et

(b) il a la capacité de libérer l'acide ribonucléique de noyaux cellulaires, caractérisé en ce que l'antigène présente les autres caractéristiques suivantes :

(c) il n'est pas précipité par une solution de sulfate d'ammonium aqueuse saturée à 30 %, à 25°C,

(d) il est précipité d'une solution aqueuse par du sulfate de streptomycine à 3,3 %,

(e) il n'a sensiblement pas d'activité d'autophosphorylation, mais il est phosphorylé par du triphosphate d'adénosine en présence d'une kinase de protéine exogène,

(f) il a la capacité de libérer l'acide ribonucléique de noyaux cellulaires,

(g) il n'est pas présent dans le sang maternel de mammifères normaux non-cancéreux en période de gestation, appartenant à l'espèce pour laquelle la présence de ladite protéine est déterminée.

2. Procédé selon la revendication 1, caractérisé en ce que la présence du produit réactionnel est déterminée par dosage radioimmunologique.

3. Procédé selon la revendication 1, caractérisé en ce que la présence du produit réactionnel est déterminée par un test ELISA.

4. Procédé selon l'une quelconque des revendications précédentes, caractérisé en ce que la matière biologique est le plasma du mammifère.

5. Procédé selon l'une quelconque des revendications 1 à 3, caractérisé en ce que les anticorps sont dérivés d'un second mammifère immunisé par l'antigène.

6. Préparation d'anticorps sensiblement dépourvue d'anticorps vis-à-vis de la fraction de plasma normale, caractérisée en ce qu'elle est précipitée par une solution de sulfate d'ammonium aqueuse saturée à 35 %, et en ce qu'elle peut former un conjugué avec une phosphoprotéine, associée au cancer, dérivée d'un mammifère et ayant les caractéristiques suivantes :

(a) elle n'est pas précipitée par une solution de sulfate d'ammonium aqueuse saturée à 30 %, à 25°C,

(b) elle a un poids moléculaire d'approximativement 60 000,

(c) elle est précipitée d'une solution aqueuse par du sulfate de streptomycine à 3,3 %,

(d) elle n'a sensiblement pas d'activité d'autophosphorylation, mais elle est phosphorylée par du triphosphate d'adénosine en présence d'une kinase de protéine exogène,

(e) elle n'a sensiblement pas d'activité d'une Kinase de protéine,

(f) elle a la capacité de libérer l'acide ribonucléique de noyaux cellulaires,

(g) elle est sensiblement dépourvue d'albumine, et

(h) elle est absente du sang maternel de mammifères normaux non-cancéreux en période de gestation, appartenant à l'espèce à partir de laquelle la protéine est dérivée,

la préparation d'anticorps formant un précipité visible avec la phosphoprotéine associée au cancer, quand elles sont diffusées l'une vers l'autre dans du gel d'agar, mais la préparation d'anticorps ne pouvant pas former un conjugué avec la fraction de protéine 25K provenant du plasma humain, ni avec la fraction de protéine 35K provenant du plasma de rat.

7. Préparation de protéine comprenant une phosphoprotéine, associée au cancer, dérivée d'un mammifère et ayant les caractéristiques suivantes :

(a) elle a un poids moléculaire d'approximativement 60 000,

(b) elle a la capacité de libérer l'acide ribonucléique de noyaux cellulaires, caractérisée en ce que la phosphoprotéine présente les autres caractéristiques suivantes :

(c) elle n'est pas précipitée par une solution de sulfate d'ammonium aqueuse saturée à 30 %, à 25°C,

(d) elle est précipitée d'une solution aqueuse par du sulfate de streptomycine à 3, 3 %,

(e) elle n'a sensiblement pas d'activité d'autophosphorylation mais elle est phosphorylée par du triphosphate d'adénosine en présence d'une kinase de protéine exogène,

(f) elle n'a sensiblement pas d'activité d'une kinase de protéine,

(g) elle est sensiblement dépourvue d'albumine, et

(h) elle est absente du sang maternel de mammifères non-cancéreux en période de gestation, appartenant à l'espèce à partir de laquelle la protéine est dérivée,

la préparation de protéine ayant une activité de libération d'ARN d'au moins environ 10 unités par milligramme de protéine totale dans la préparation de protéine.

**Patentansprüche**

1. Eine Methode zur Bestimmung des Vorhandenseins eines Krebs-assoziierten Antigens in einem Säugetier, wobei die Methode umfaßt:

die Produktion von Antikörpern gegen das Antigen;

das in Kontakt bringen der Antikörper mit dem

biologischen Material des Säugetiers, und

Bestimmung des Vorhandenseins eines Reaktionsprodukts, welches aufgrund des Zusammenwirkens der Antikörper und dem Antigen gebildet wird, wobei das Antigen, welches ein Phosphoprotein sei, folgende Eigenschaften hat:

(a) es hat ein Molekulargewicht von ungefähr 60000; und

(b) es besitzt die Eigenschaft Ribonucleinsäure aus dem Zellkern freizusetzen,

**dadurch gekennzeichnet**, daß das Antigen die weiteren folgenden Eigenschaften hat:

(c) es wird durch eine 30% gesättigte wässrige Ammoniumsulfatlösung bei 25° C nicht gefällt;

(d) es wird aus wässriger Lösung mit 3.3% Streptomycinsulfat gefällt;

(e) es hat substantiell keine autophosphorylierende Aktivität, wird aber durch Adenosintriphosphat in Gegenwart einer exogenen Proteinkinase phosphoryliert;

(f) es besitzt die Eigenschaft Ribonucleinsäure aus dem Zellkern freizusetzen;

(g) es ist nicht vorhanden im mütterlichen Blut von nicht krebskranken normal trächtigen Säugetieren, welche von der Spezies sind in der besagtes Protein bestimmt wird.

2. Eine Methode nach Anspruch 1, **dadurch gekennzeichnet**, daß die Anwesenheit des Reaktionsproduktes vermittels Radioimmunoassay bestimmt wird.

3. Eine Methode nach Anspruch 1, **dadurch gekennzeichnet**, daß die Anwesenheit des Reaktionsproduktes durch ELISA Assay bestimmt wird.

4. Eine Methode nach irgendeinem der vorherigen Ansprüche,
**dadurch gekennzeichnet**, daß das biologische Material aus Säugetierplasma besteht.

5. Eine Methode nach irgendeinem der Ansprüche 1 bis 3,
**dadurch gekennzeichnet**, daß die Antikörper von einem zweiten Säugetier stammen, welches mit dem Antigen immunisiert wurde.

6. Eine Antikörperpräparation, welche substantiell frei von Antikörpern gegen eine normale Plasmafraktion ist, wobei diese Antikörperpräparation **dadurch gekennzeichnet** ist, daß sie durch 35% gesättigtes wässriges Ammoniumsulfat ausgefällt wird und **dadurch gekennzeichnet**, daß sie die Eigenschaft besitzt ein Konjugat mit einem Krebs-assoziierten Phosphoprotein zu bilden, welches von einem Säugetier stammt und folgende Eigenschaften besitzt:

(a) es wird durch eine 30% gesättigte wässrige Ammoniumsulfatlösung bei 25°C nicht gefällt;

(b) es besitzt ein Molekulargewicht von ungefähr 60000;

(c) es wird aus einer wässrigen Lösung mit 3.3% Streptomycinsulfat gefällt;

d) es hat substantiell keine autophosphorylierende Aktivität, aber es wird mit Adenosintriphosphat in der Gegenwart einer exogenen Proteinkinase phosphoryliert;

(e) es hat substantiell keine Aktivität einer Proteinkinase;

(f) es hat die Eigenschaft Ribonucleinsäure aus dem Zellkern freizusetzen;

(g) es ist substantiell frei von Albumin; und

(h) getrennt vom mütterlichen Blut von nicht krebskranken, trächtigen Säugetieren, welche von der Spezies sind von der besagtes Protein stammt, bildet die Antikörperpräparation ein sichtbares Präzipitat mit dem Krebs-assoziierten Phosphoprotein, wenn sie zusammen in einem Agargel vermischt werden, wobei die Antikörperpräparation weder die Eigenschaft besitzt mit der 25K Proteinfraktion aus Humanplasma ein Konjugat zu bilden noch die Eigenschaft besitzt mit der 35K Proteinfraktion aus Rattenplasma ein Konjugat zu bilden.

7. Eine Proteinpräparation, welche ein Krebs-assoziiertes Phosphoprotein umfaßt, wobei dieses von einem Säugetier stammt und folgende Eigenschaften besitzt:

(a) es hat ein Molekulargewicht von ungefähr 60000; und

(b) es hat die Eigenschaft Ribonucleinsäure aus dem Zellkern freizusetzen,
**dadurch gekennzeichnet**, daß das Phosphoprotein die folgenden weiteren Eigenschaften besitzt:

(c) es wird nicht mit 30% gesättigter wässriger Ammoniumsulfatlösung bei 25° gefällt;

(d) es wird aus einer wässrigen Lösung mit 3.3% Streptomycinsulfat gefällt

(e) es hat substantiell keine autophosphoylierende Aktivität, aber es wird mit Adenosintriphosphat in der Gegenwart einer exogenen Proteinkinase phosphoryliert;

(f) es hat substantiell keine Aktivität einer Proteinkinase;

(g) es ist substantiell freivon Albumin; und

(h) getrennt vom mütterlichen Blut von nicht krebskranken, trächtigen Säugetieren, welche von der Spezies sind von der besagtes Protein stammt, hat die Proteinpräperation eine RNA-Freisetzungsaktivität von wenigstens um 10 Units pro Milligram Totalprotein in der Proteinpräparation.

FIG. 1

FIG. 2